# EUROPEAN PATENT SPECIFICATION

(11) **EP 1 841 778 B1**
(45) Date of publication and mention of the grant of the patent: **14.03.2012**
(21) Application number: 06700710.4
(22) Date of filing: 17.01.2006
(51) Int. Cl.: C07J 7/00

(54) **METHOD FOR PREPARING MEDROGESTONE**
VERFAHREN ZUR HERSTELLUNG VON MEDROGESTON
PROCÉDÉ POUR LA PRÉPARATION DE MEDROGESTONE

(30) Priority: 18.01.2005 US 644053 P; 18.01.2005 EP 05100267
(43) Date of publication of application: 10.10.2007
(73) Proprietor: Abbott Products GmbH, 30173 Hannover (DE)
(72) Inventor: MOUTOU, Jean-luc, F-06800 Cagnes Sur Mer (FR); RONDOT, Benoît, F-06480 La Colle Sur Loup (FR); LAFAY, Jean, F-06100 Nice (FR); MAILLOS, Philippe, 30173 Hannover (DE)
(74) Representative: Hubert, Philippe
(86) International application number: PCT/EP2006/050242
(87) International publication number: WO 2006/077209

(56) References cited:
- EP-A- 0 085 900
- EP-A- 0 680 970
- GB-A- 1 065 189
- US-A- 3 170 936
- D. BURN ET AL: "Modified Steroid Hormones - XXXVIII Some transformations of steroidal 3-Alkoxy-6-formyl-3,5-dienes and related compounds" TETRAHEDRON., vol. 21, 1964, pages 1619-1624, XP002333826 NLELSEVIER SCIENCE PUBLISHERS, AMSTERDAM. cited in the application
- F. SCHNEIDER ET AL: "Über den Verlauf der Umsetzung von Steroid-3,5-dienaniminen mit Formaldehyd" HELVETICA CHIMICA ACTA., vol. 56, no. 7, 1973, pages 2396-2404, XP002333827 CHVERLAG HELVETICA CHIMICA ACTA. BASEL.

## Description

The present invention relates to a method for the preparation of medrogestone by heterogeneously palladium-catalysed isomerisation from 17α-methyl-6-methylenepregn-4-ene-3,20-dione.

Medrogestone is a 6-methyl steroid which is used e.g. for the treatment of certain disorders in women's menstrual flow or to supplement oestrogen treatment in the climacteric period.

US Patent 3,170,936 describes a method for the preparation of medrogestone by acid-catalysed elimination of water from 5α,6β-dihydroxy-6α-17α-dimethylpregnan-3,20-dione.

A method for the preparation of, inter alia, medrogestone is known from Patent EP 0 085 900 B1, wherein a corresponding Δ⁴-3-ketosteroid is reacted with e.g. methoxymethyl acetate in the presence of catalytic amounts of phosphorus oxychloride and an alkali metal acetate.

Patent EP 0 680 970 B1 discloses a method for the preparation of medrogestone from 6β-hydroxy-6α,17α-dimethylpregn-4-ene-3,20-dione by reaction with boron trifluoride etherate. 17α-methyl-6-methylenepregn-4-ene-3,20-dione produced as byproduct is regarded as disruptive in this known method and has to be removed by reaction with maleic anhydride in order to obtain pure medrogestone.

Methods for isomerisation of a 6-methylene-4-en-3-one system on different steroid compounds for the preparation of the biologically valuable 6-methyl-4,6-dienone system are known for example from D. Burn et al., Tetrahedron 21 (1965) 1619-1624 or from F. Schneider et al., Helv. Chim. Acta 56/7 (1973) 2396-2404, but in the methods given therein the addition of a hydrogen donor is deemed obligatory when performing the heterogeneously catalysed isomerisation reaction. Further isomerisation reactions are for example known from German Offenlegungsschrift DE-OS 25 22 533 (= GB 1 515 284) and from US Patent 4,544,555. Furthermore, the steroid systems listed above as examples contain no methyl group in position 17 of the steroid structure. It is assumed of substituents in position 17 of the steroid structure that they considerably reduce the reactivity of a steroid derivative for an isomerisation reaction by steric screening (cf. e.g. L. F. Fieser and M. Fieser "Steroide", Verlag Chemie, Weinheim/Bergstr. 1961, pp. 10-20), in particular if at the same time a further substituent such as a methyl group is located in position 18 of the steroid structure.

It was an object of the invention to provide a novel method for the preparation of medrogestone which is simple and inexpensive to perform and accordingly is also suitable for large-scale industrial synthesis.

It has now been found that medrogestone can be obtained simply and inexpensively by heterogeneously catalysed isomerisation from 17α-methyl-6-methylenepregn-4-ene-3,20-dione and purification and processing steps which can be carried out subsequently if desired. The addition of a hydrogen donor such as elemental hydrogen or cyclohexene when performing the heterogeneously catalysed isomerisation reaction is not necessary according to the method in accordance with the present invention.

The subject of the invention is therefore a method for the preparation of medrogestone of Formula I, 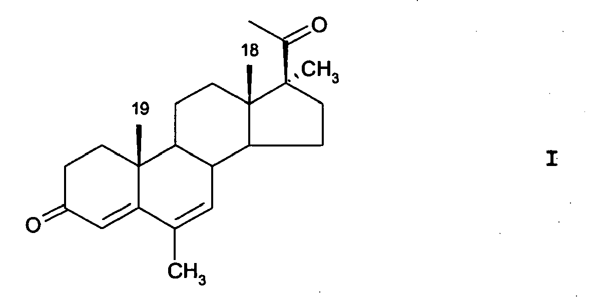 wherein 17α-methyl-6-methylenepregn-4-ene-3,20-dione of Formula II 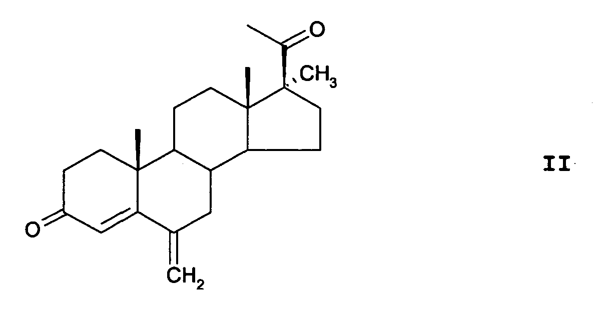 is isomerised in a C₁₋₄ alcohol or in a mixture of such C₁₋₄ alcohols and in the presence of a supported palladium catalyst at a temperature of 60°C to 95°C and wherein no hydrogen donor is added to form a compound of Formula I.

The method is suitable for the preparation of medrogestcolne in large-scale industrial synthesis. For example, approximately 5 - 100 kg, preferably 25 - 60 kg, of the starting compound of Formula II can be used. The yield in that case, dependent on, inter alia, the number of purification stages, is up to 95%, relative to the mass of starting compound of Formula II used.

C₁₋₄ alcohols suitable as solvents or solvent mixtures may be straight-chain or branched. Ethanol, methanol, 2-propanol and mixtures of these aforementioned alcohols are preferred solvents. Ethanol, in particular absolute ethanol, is particularly preferred. The solvent or solvent mixture is usually calculated in a ratio of 8 - 15 volume units, relative to one mass unit of starting compound of Formula II. A volume unit (= VU) in the context of the present invention is calculated in litres. A mass unit (= MU) in the context of the present invention is calculated in kilogrammes.

In particular palladium on aluminium oxide (= Pd/Al₂O₃), palladium on activated carbon (= Pd/C) and/or palladium on calcium carbonate can be used as supported palladium catalysts. Usually 5% Pd/C or 5% Pd/Al₂O₃ is used. 5% Pd/C is preferred. The supported palladium catalyst is usually calculated in a ratio of 0.15 - 0.25 MU, relative to one MU of starting compound of Formula II.

The reaction is carried out at a temperature of 60°C to 95°C, usually at the boiling temperature of the solvent or solvent mixture. Preferably the reaction temperature is greater than or equal to 72°C, in order to avoid the formation of byproducts as far as possible. In a particularly preferred variant, the reaction is carried out at the boiling temperature of ethanol, in particular denatured ethanol.

Usually the supported palladium catalyst and the solvent are placed in a suitable reaction vessel such as a suitably sized stainless steel container under a protective gas atmosphere and with moisture excluded and heated under reflux cooling to a temperature of 60°C to 95°C, in particular to boiling, before the starting compound of Formula II is added to this initial solution. The reaction is then continued under reflux cooling at boiling heat, until as complete a reaction as possible has occurred. The progress of the reaction can be followed in known manner, for example by means of high-performance liquid chromatography (= HPLC) or by means of thin-layer chromatography (= TLC). Usual reaction times are 1.5 - 3 hours (= h).

Then the medrogestone prepared can be isolated and/or purified if desired.

To isolate medrogestone, in a first variant the reaction mixture can be cooled to approx. 35 to 50°C and be filtered through a known filter. Then the solvent can be largely evaporated, for example to approximately one third of the starting volume, from the filtrate at reduced pressure in known manner. The remaining residue can then be cooled for approx. 1 - 2 h to temperatures of 0°C to 5°C, with medrogestone precipitating as a solid, preferably in crystalline form. The resulting solid medrogestone can then also be dried in known manner, for example at reduced pressure and a temperature of 40°C for a duration of approximately 6 - 10 h.

To isolate medrogestone, in a second variant the reaction mixture can be filtered at a temperature of 65°C to 75°C in known manner through a suitable filter and the volume of the filtrate can then be evaporated at reduced pressure in known manner to approx. 5.5 - 6.5 VU, relative to one MU of starting compound of Formula II. The resulting filtrate of reduced volume can then be heated under reflux cooling to a temperature above room temperature, in particular to boiling, before 3.5 - 4.5 VU water, relative to one MU of starting compound of Formula II, are added at this elevated temperature. Once the water has been added, the aqueous alcohol solution can then be cooled to a temperature of 0°C to 15°C. It is particularly advantageous to cool the reaction solution in steps, in particular to remain at the elevated starting temperature for 5 - 10 minutes (= min.) after the addition of the water, then to cool it to 18 to 24°C for 25 - 35 min. and finally to cool it for 50 - 70 min. to 0°C to 15°C, in particular to 10°C, with medrogestone precipitating as solid, preferably in crystalline form. The resulting solid medrogestone can then if desired also be dried in known manner, for example at reduced pressure and a temperature of 20°C to 40°C for a duration of approximately 6 h - 36 h.

For purification of medrogestone, in a first variant the solid medrogestone obtained for isolation according to the first or second variant can be dissolved in a suitable inert polar organic solvent or a solvent mixture at a temperature above room temperature, in particular at the boiling temperature of the solvent or solvent mixture, and then can be precipitated again by cooling to room temperature or to a temperature below room temperature. In this variant methanol, ethanol, 2-propanol and mixtures thereof can be used as solvent. In particular ethanol can be used, preferably 3.5 - 4.5 volume units of ethanol, relative to one MU of solid medrogestone obtained according to the first or second variant. For example, one MU of medrogestone can be added to 3.5 - 4.5 VU ethanol and be heated to boiling for 5 - 15 min. under reflux cooling. Then the batch can be cooled to 5°C to 15°C and be kept at this temperature for 25 min. - 35 min. before being cooled to 5°C and maintained for 45 min. to 90 min. at this temperature. The resulting crystals of medrogestone can then be filtered off and dried in known manner.

For purification of medrogestone, in a second variant the solid medrogestone obtained for isolation according to the first or second variant or the crystals of medrogestone obtained for purification according to the first variant can be dissolved in a suitable inert polar organic solvent or a solvent mixture at a temperature above room temperature, in particular at the boiling temperature of the solvent or solvent mixture, and then be precipitated again by cooling to room temperature or to a temperature below room temperature. In this variant in particular 2-propanol can be used as solvent, preferably 3.5 - 4.5 VU 2-propanol, relative to one MU of solid medrogestone obtained according to the first or second variant or crystals of medrogestone obtained for purification according to the first variant. In particular, one MU of medrogestone can be added to 3.5 - 4.5 VU of 2-propanol and heated to boiling under reflux cooling. Once the medrogestone has completely dissolved, the batch is filtered in known manner and the resulting filtrate is cooled, preferably at a rate of 2°C/min. It is beneficial during the cooling operation to seed the batch with small amounts of crystalline medrogestone. The resulting crystals of medrogestone can then be washed with 2-propanol (preferably with 0.4 to 0.6 VU, relative to one MU of medrogestone solid used) and in known manner filtered off and dried to a desired specification.

The solid medrogestone obtained according to one of the isolation and/or purification methods described above can then also be micronised in known manner.

The compound of Formula II can be obtained by first reacting a compound of the general Formula III, 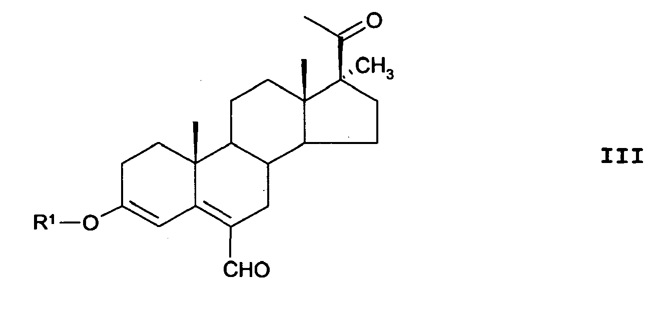 wherein R¹ is C₁₋₄ alkyl, in a polar organic solvent which is inert under the reaction conditions or in a solvent mixture with an alkali metal hydride and the resulting intermediate is then reacted with a reagent suitable for cleaving the C₁₋₄ alkyl ether. In the context of the present compounds, C₁₋₄ alkyl groups may be branched or unbranched. Ethyl is preferred as C₁₋₄ alkyl group.

Suitable solvents or solvent mixtures are for example methanol; ethanol; 1-propanol; 2-propanol; tert. butanol; 2-butanol; 1-butanol; THF; 1,4-dioxane; acetone and also mixtures of the aforementioned solvents. Ethanol, THF, acetone and mixtures thereof are preferred. The solvent or solvent mixture is usually calculated in a ratio of 6 - 10 VU, relative to one MU of starting compound of Formula III.

Suitable alkali metal hydrides are for example LiAIH₄, NaBH₄, NaBH₃CN or Zn(BH₄)₂. NaBH₄ is preferred.

Suitable reaction temperatures are from -10°C to 0°C, preferably -5°C.

Usually one MU of starting compound of Formula III, preferably 17α-methyl-3-ethoxy-6-formylpregna-3,5-dien-20-one, is dissolved or suspended in 3.5 - 4.5 VU solvent or solvent mixture, in particular ethanol, relative to one MU of starting compound of Formula III, and it is cooled to an internal temperature of the reaction mixture of 5°C to 15°C. 0.02 - 0.04 MU alkali metal hydride, preferably NaBH₄, are added to this initial solution, followed by 0.04 - 0.06 VU acetone, and 3.5 - 4.5 VU THF, each relative to one MU of starting compound of Formula III. Then it is cooled further to an internal temperature of the reaction mixture of -10°C to 0°C, preferably -5°C. Then the C₁₋₄ alkyl ether can be acid-cleaved, preferably in a one-pot reaction, for example by addition of a dilute aqueous sulphuric acid solution (usually 0.1 VU sulphuric acid and 0.1 VU water, each relative to one MU of starting compound of Formula III). Once the reaction has taken place, the reaction mixture can be neutralised with an alkali metal carbonate such as potassium carbonate or an aqueous solution of the alkali metal carbonate. The resulting product of Formula II can then be precipitated by addition of water to the reaction mixture and be isolated and purified in known manner.

Compounds of Formula III can be obtained by reacting a compound of Formula IV, 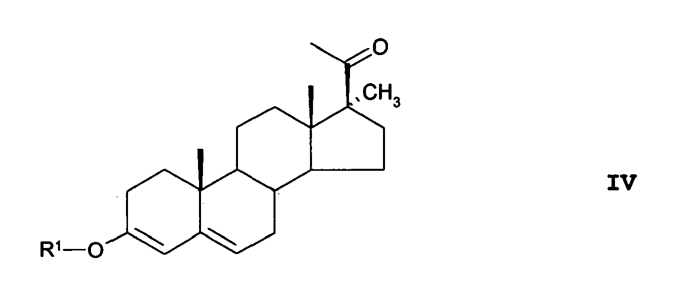 wherein R¹ has the above meaning, in an organic solvent which is inert under the reaction conditions or in a solvent mixture with an N-disubstituted formamide, preferably DMF, and phosphorus oxychloride.

The reaction can be carried out under the known conditions of what is called a Vilsmeier formylation, the starting compound of Formula IV being reacted with 0.20 - 0.35 VU, preferably 0.28 MU, DMF, relative to one MU of starting compound of Formula IV, and 0.45 - 0.50 MU, preferably 0.473 MU, phosphorus oxychloride, relative to one MU of starting compound of Formula IV.

Suitable solvents or solvent mixtures are open-chain or cyclic di-lower alkyl ethers such as diethyl ether; diisopropyl ether; THF; 1,4-dioxane, and also mixtures of the aforementioned solvents. DMF can also be used as solvent. THF is preferred as solvent. The solvent or solvent mixture is usually calculated in a ratio of 1.5 - 6 VU, preferably 1.5 - 2.5 VU, relative to one MU of starting compound of Formula IV.

Suitable reaction temperatures are -25°C to -5°C, preferably approximately -15°C.

It is beneficial to carry out the reaction in the presence of at least catalytic amounts of a non-nucleophilic organic base such as a nitrogen base, for example triethylamine.

Once the reaction is completed, an amount, sufficient for neutralisation, of a base such as an alkali metal carbonate base, for example potassium carbonate, or an aqueous solution of an alkali carbonate base is added to the reaction mixture with cooling to -10°C to 10°C. For example, a solution of 0.8 - 0.9 MU, preferably 0.853 MU, potassium carbonate, in 1.5 - 2 VU, preferably in 2 VU, water, in each case relative to one MU of starting compound of Formula IV, can be added.

The resulting compound of Formula III can then also be purified and/or isolated. To this end, the batch, after thawing to 15°C to 25°C, can first be diluted with 2.5 - 3.5 VU ethanol, relative to one MU of starting compound of Formula IV, and stirred for 20 - 40 min.. Then the batch can be diluted with 9 - 11 VU water, relative to one MU of starting compound of Formula IV, and stirred for a further 20 - 90 min. The resulting precipitate can then be filtered off, subsequently washed with 1.5 - 2.5 VU water, relative to one MU of starting compound of Formula IV, and partially dried, for example in a vacuum for 1 - 8 h. Then the initially dried precipitate can be stirred with 4 - 6 VU water, relative to one MU of starting compound of Formula IV, and an at least catalytic amount of a non-nucleophilic organic base such as a nitrogen base, preferably with 0.04 - 0.06 VU triethylamine, relative to one MU of starting compound of Formula IV, at a temperature of 15°C to 30°C for 30 - 120 min. Then the precipitate can be filtered off again and washed with water until no chloride ions are indicated in the filtrate. For purification, the compound of Formula III resulting as precipitate can be heated to a temperature above room temperature, preferably under reflux cooling and to boiling temperature of the solvent, with 1.5 - 2.5 VU ethanol, relative to one MU of starting compound of Formula IV, and an at least catalytic amount of a non-nucleophilic organic base such as a nitrogen base, preferably with 0.01 - 0.03 VU triethylamine, relative to one MU of starting compound of Formula IV, until the compound of Formula III dissolves. After cooling to room temperature, the resulting recrystallised compound of Formula III can be washed with 0.4 - 0.6 VU ethanol, relative to one MU of starting compound of Formula IV, and dried to a desired specification. Yields of 68 - 85% of the compound of Formula III are obtained, relative to the starting compound of Formula IV which is used.

Compounds of the general formula IV can be prepared by alkylating an enol ether of the general formula V, 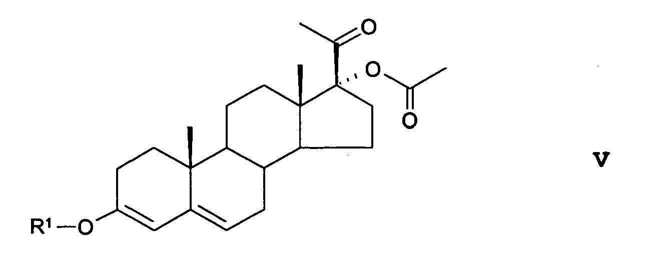 wherein R¹ has the above meaning, in known manner under the conditions of what is called a Birch reduction with lithium in liquid ammonia with a methyl halide, preferably methyl iodide.

Compounds of Formula V are known per se, for example from US Patent 3,240,671, and can be prepared according to the methods disclosed therein, analogous methods, or other methods. For example, compounds of Formula V can be prepared by reacting known 17α-acetoxyprogesterone in known manner with a suitable orthoester.

### Example 1:

### 6,17α-dimethylpregna-4,6-diene-3,20-dione (medrogestone, compound of Formula I)

A) 100 kg 17α-acetoxyprogesterone (VI) is dissolved in 400 litres (= I) absolute ethanol. 100 l triethyl orthoformate and 500 g p-toluenesulphonic acid are added thereto. The mixture is heated for 4 h to 35°C, allowed to cool to room temperature (= RT), 7.26 g triethylamine is added and the mixture is cooled to 0°C for 1 h. The resulting solid is filtered and washed at 0°C with 50 l cold ethanol containing 1 vol. % triethylamine. The washed solid is left under reflux cooling and at boiling heat in 800 l diisopropyl ether containing 1 vol. % triethylamine until the solid is dissolved. Then approx. 200 l diisopropyl ether is distilled off, the solution is cooled to 0°C for 1 h and the 17α-acetoxy-3-ethoxy-3,5-pregnadien-20-one (V) occurring as solid is filtered and dried at temperatures below 40°C and at reduced pressure for 6 h.
B) 3.58 kg lithium is added at -70°C to 640 l liquid ammonia over 15 min.. After 30 min., a solution of 80 kg of an ethyl enol ether as obtained above in 1280 l THF is added for 2 h, the internal temperature being kept at approximately -60°C to -65°C. Once addition is complete, the reaction mixture is kept for 1 h at -65°C, then 74.4 l methyl iodide is added over 15 min.. The temperature is maintained at -65°C for a further 2 h and then the ammonia is evaporated off. THF is distilled off and the remaining residue is taken up with 320 l toluene. Undissolved solids are filtered off and the mixture is subsequently washed with 100 l toluene. 160 l ethyl acetate is added to the filtrate and the organic phase is washed twice with water. The solvent is evaporated and a solid is obtained which is recrystallised from 400 l methanol containing 1 vol. % triethylamine. Cooling of the mother liquor for 1 h to 0°C yields 51.3 kg 17α-methyl-3-ethoxy-3,5-pregnadien-20-one (IV) as a white crystalline powder of a melting point of 114 to 116°C.
C) 70 kg of a 17α-methyl-3-ethoxy-3,5-pregnadien-20-one as obtained above is suspended in 140 l THF in an enamelled reaction vessel and 700 g triethylamine and 19.6 l DMF are added. This initial solution is cooled to -15°C before 33.1 kg phosphorus oxychloride is added. The reaction is continued until complete reaction has taken place (HPLC monitoring), then a cooled solution of 59.7 kg potassium carbonate in 140 l purified water is added, stirred for 1 h and allowed to thaw to 20°C. 210 l denatured ethanol is added, the mixture is stirred for 30 min., 700 l purified water is added and the mixture is stirred for a further 60 min.. The resulting solid is vacuum-filtered, subsequently washed with a total of 140 l purified water and dried on the filter for 8 h in a vacuum. Then it is taken up with 350 l purified water, 3.5 l triethylamine is added and the mixture is stirred for 1 h at room temperature. Filtration takes place in a vacuum, the solid residue is washed with purified water until no chloride ions can be detected, and then dried for 8 h in a vacuum at 40°C. Finally the solid is taken up in 140 l denatured ethanol, 1.4 l triethylamine is added thereto and the mixture is heated to boiling under reflux cooling, until dissolving of the solid is observed. After cooling to room temperature, the 17α-methyl-6-formyl-3-ethoxy-3,5-pregnadien-20-one (III) which again results is filtered off from the mother liquor, subsequently washed with 35 l purified water and then dried for 8 h in a vacuum at 40°C. The yield is 87.5%, relative to the 17α-methyl-3-ethoxy-3,5-pregnadien-20-one used.
D) 60 kg of a 7α-methyl-6-formyl-3-ethoxy-3,5-pregnadien-20-one as obtained above is suspended in 240 l denatured ethanol in a stainless steel reaction vessel and cooled until the internal temperature of the suspension is 10°C. 1.8 kg NaBH₄ is added to this initial solution and stirring is continued until complete reaction has occurred (HPLC monitoring). 3 l acetone and 240 l THF are added and the batch is cooled to -5°C. Then a solution of 6 l sulphuric acid in 6 l purified water is added and is stirred for 40 min., before a solution of 30 kg potassium carbonate in 66 l purified water is added. Stirring is continued until a pH of 7 is set, then 900 l purified water is added, the resulting suspension is stirred, the resulting precipitate is filtered under vacuum and partially dried. The initially dried precipitate is taken up in 240 l purified water, stirred at room temperature and filtered in a vacuum. The solid is washed with purified water until no sulphate ions are detected and the pH value of the washing water is neutral. The washed solid is then dried for 8 h in a vacuum at 40°C, is then taken up in 200 l denatured ethanol and heated under reflux cooling until the solid is dissolved as completely as possible. Then it is cooled to room temperature, the resulting precipitate is filtered off, subsequently washed with 20 l denatured ethanol and dried in a vacuum. The initially dried precipitate is taken up in 76 l THF and heated under reflux cooling and with stirring until the solid is dissolved as completely as possible. Then it is cooled to room temperature, the resulting precipitate is filtered off and subsequently washed with 19 l THF. Then the resulting 17α-methyl-6-methylenepregn-4-ene-3,20-dione (II) is dried in a vacuum for 8 h at 40°C. The yield is 62.7%, relative to the α-methyl-6-formyl-3-ethoxy-3,5-pregnadien-20-one used.
E) 6 kg of 5% Pd/C catalyst is suspended in 297 l absolute ethanol in a stainless steel reaction vessel and heated to boiling with stirring and under reflux cooling. 30 kg of a 17α-methyl-6-methylenepregn-4-ene-3,20-dione as obtained above is added to this initial solution, and stirring is continued until complete reaction has occurred (HPLC monitoring). Then the catalyst is filtered off, it is subsequently washed with 120 l absolute ethanol and the organic phase is evaporated to a volume of approximately 180 l. 120 l purified water is added to the concentrated solution and the mixture is cooled to room temperature. The resulting crystals are filtered off under a nitrogen atmosphere, washed in succession with 9 l absolute ethanol and 6 l purified water and then dried for 8 h in a vacuum at 40°C.
   The dried precipitate is taken up in 100 l absolute ethanol and heated to boiling with stirring and under reflux cooling, until the solid is dissolved as completely as possible. Then it is cooled to room temperature, the resulting crystals are vacuum-filtered under nitrogen atmosphere, subsequently washed with 12.5 l absolute ethanol and the resulting medrogestone (I) is dried in a vacuum at 40°C. The yield is 80.5%, relative to the 17α-methyl-6-methylenepregn-4-ene-3,20-dione used.
F) 25 kg medrogestone as obtained above is suspended in 100 l 2-propanol in a stainless steel reaction vessel and heated to boiling with stirring and under reflux cooling, until the solid is dissolved as completely as possible. The resulting solution is filtered and cooled to room temperature at a rate of 2°C/min., the mother liquor being initially seeded with medrogestone seed crystals. The resulting crystals of recrystallised medrogestone are vacuum-filtered under nitrogen atmosphere and subsequently washed with 12.5 l 2-propanol. Then the medrogestone crystals are dried at 40°C in a vacuum. The yield is 88.0%, relative to the medrogestone used before recrystallisation.

The dried, recrystallised medrogestone crystals thus obtained are then micronised to a defined particle size in known manner under nitrogen atmosphere. The micronised medrogestone is then packaged in polyethylene bags.

### Example 2:

### 6,17α-dimethylpregna-4,6-diene-3,20-dione (medrogestone, compound of Formula I) - alternative synthesis

5% Pd/Al₂O₃ (29.25 g) was heated to boiling in abs. ethanol (1.17 l) which contained 1% (v/v) purified water, under reflux cooling. 17α-methyl-6-methylenepregn-4-ene-3,20-dione was quickly added to this initial solution (117 g, for preparation see Example 1 D)) in powder form. During the addition the temperature was kept at 75°C. The reflux cooling was maintained for 30 min., then the heterogeneous catalyst was filtered off, subsequently washed with hot ethanol (470 ml) and the filtrate was concentrated to approx. 800 ml at reduced pressure. Then water was added until the crystalline medrogestone precipitated, which was filtered and subsequently dried under nitrogen atmosphere. 108.8 g crystalline medrogestone was obtained.

## Claims

1. A method for the preparation of medrogestone of Formula I, 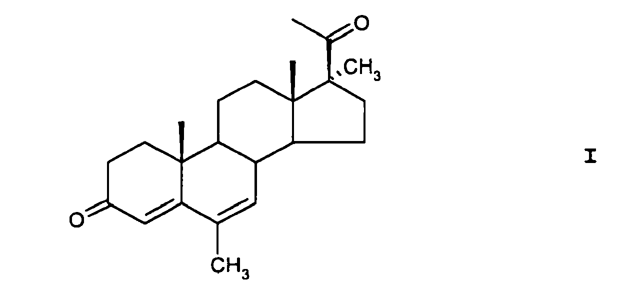 **characterised in that** a compound of Formula II 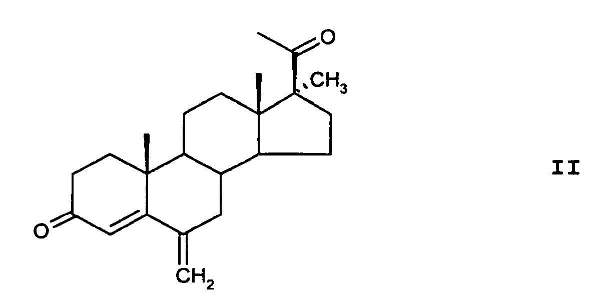 is isomerised in a C₁₋₄ alcohol or in a mixture of such C₁₋₄ alcohols and in the presence of a supported palladium catalyst at a temperature of 60°C to 95°C and wherein no hydrogen donor is added to form a compound of Formula I.

2. A method according to Claim 1, which is carried out in methanol, ethanol, 2-propanol or in a mixture of the aforementioned alcohols.

3. A method according to Claim 1, which is carried out in ethanol.

4. A method according to Claim 1, wherein palladium on aluminium oxide, palladium on activated carbon and/or palladium on calcium carbonate is used as supported palladium catalyst.

5. A method according to Claim 4, wherein 5% palladium on activated carbon is used as supported palladium catalyst.

6. A method according to Claim 1, wherein the method is carried out at a temperature greater than or equal to 72°C.

7. A method according to Claim 1, wherein the medrogestone obtained after the isomerisation is precipitated as solid, preferably in crystalline form, after the addition of water.

8. A method according to Claim 7, wherein the medrogestone precipitated as solid is dissolved at a temperature above room temperature by addition of 3.5 - 4.5 volume units of ethanol, relative to one mass unit of medrogestone precipitated as solid, and then is precipitated again by cooling to room temperature or a temperature below room temperature.

9. A method according to Claim 7 or 8, wherein the medrogestone obtained as solid in each case after precipitation is further purified by recrystallisation from 2-propanol.

10. A method according to one of Claims 7-9, wherein the medrogestone obtained as solid in each case is then micronised.

11. A method according to Claim 1, **characterised in that** the compound of Formula II is obtained by first reducing a compound of Formula III, 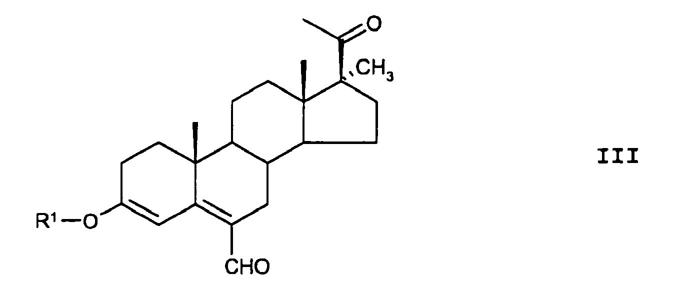 wherein R' is C₁₋₄ alkyl, in a polar organic solvent which is inert under the reaction conditions or in a solvent mixture with an alkali metal hydride and the resulting intermediate is then reacted with a reagent suitable for cleaving the C₁₋₄ alkyl ether.

12. A method according to Claim 11, **characterised in that** the compound of Formula III is obtained by reacting a compound of Formula IV, 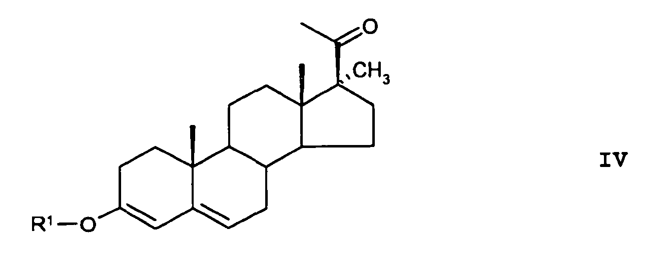 wherein R¹ is C₁₋₄ alkyl, in an organic solvent which is inert under the reaction conditions or in a solvent mixture with dimethyl formamide and phosphorus oxychloride.

## Patentansprüche

1. Verfahren zur Herstellung von Medrogeston mit der Formel I, 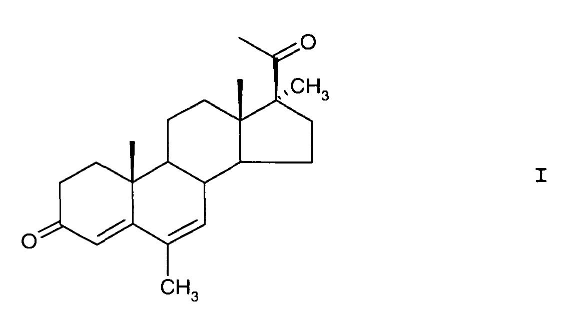 **dadurch gekennzeichnet, daß** eine Verbindung mit Formel II 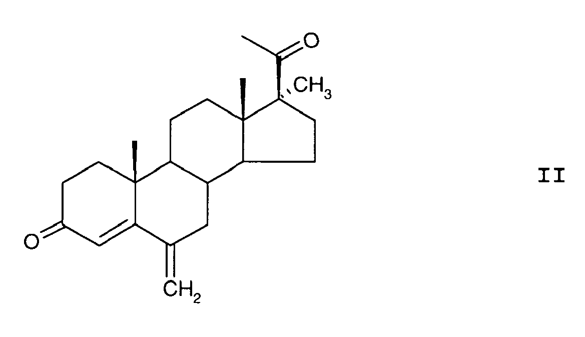 isomerisiert wird in einem C₁₋₄-Alkohol oder in einem Gemisch solcher C₁₋₄-Alkohole und in Gegenwart eines Palladiumkatalysators mit Träger bei einer Temperatur von 60°C bis 95°C und wobei kein Wasserstoffdonor zugegeben wird, um eine Verbindung mit Formel I zu bilden.

2. Verfahren nach Anspruch 1, welches in Methanol, Ethanol, 2-Propanol oder einem Gemisch der genannten Alkohole ausgeführt wird.

3. Verfahren nach Anspruch 1, welches in Ethanol ausgeführt wird.

4. Verfahren nach Anspruch 1, wobei Palladium auf Aluminiumoxyd, Palladium auf Aktivkohle und/oder Palladium auf Kalziumcarbonat verwendet wird als ein Palladiumkatalysator mit Träger.

5. Verfahren nach Anspruch 4, wobei 5% Palladium auf Aktivkohle als Palladiumkatalysator mit Träger verwendet wird.

6. Verfahren nach Anspruch 1, wobei das Verfahren bei einer Temperatur größer oder gleich 72°C ausgeführt wird.

7. Verfahren nach Anspruch 1, wobei das Medrogeston, das nach der Isomerisierung erhalten wird, als Feststoff, vorzugsweise in kristallisierter Form, nach der Zugabe von Wasser ausgefällt wird.

8. Verfahren nach Anspruch 7, wobei das Medrogeston, das als Feststoff ausgefällt ist, gelöst wird bei einer Temperatur oberhalb der Raumtemperatur durch Zugabe von 3,5 bis 4,5 Volumeneinheiten Ethanol bezüglich einer Masseneinheit von als Feststoff ausgefälltem Medrogeston und dann erneut ausgefällt wird durch Kühlen auf Raumtemperatur oder eine Temperatur unterhalb der Raumtemperatur.

9. Verfahren nach Anspruch 7 oder 8, wobei das Medrogeston, das in jedem Fall nach Ausfällen als Feststoff erhalten wird, weiter gereinigt wird durch Umkristallisation aus 2-Propanol.

10. Verfahren nach einem der Ansprüche 7 bis 9, wobei das Medrogeston, das als Feststoff in jedem Fall erhalten wird, dann mikronisiert wird.

11. Verfahren nach Anspruch 1, **dadurch gekennzeichnet, daß** die Verbindung mit Formel II erhalten wird durch zunächst Reduzieren einer Verbindung mit Formel III, 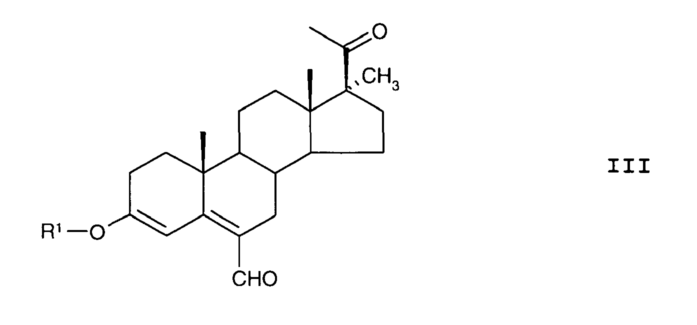 wobei R¹ ein C₁₋₄-Alklyl ist, in einem polaren organischen Lösungsmittel, welches unter den Reaktionsbedingungen inert ist, oder in einem Lösungsmittelgemisch mit einem Alkalimetallhydrid und das resultierende Intermediat dann reagiert wird mit einem Reagenz, das in der Lage ist, den C₁₋₄-Alkylether zu spalten.

12. Verfahren nach Anspruch 11, **dadurch gekennzeichnet, daß** die Verbindung mit Formel III erhalten wird durch Reagieren einer Verbindung mit Formel IV, 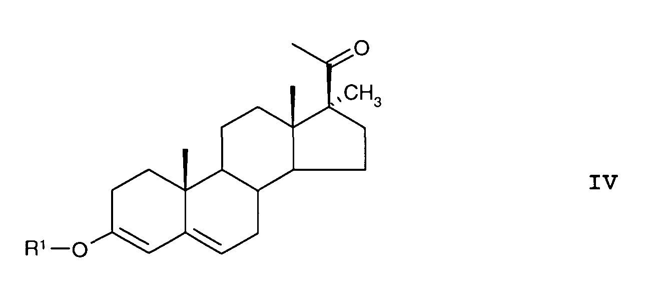 wobei R¹ ein C₁₋₄-Alkyl ist, in einem organischen Lösungsmittel, welches unter den Reaktionsbedingungen inert ist, oder in einem Lösungsmittelgemisch, mit Dimethylformamid und Phosphoroxychlorid.

## Revendications

1. Procédé pour la préparation de médrogestone de formule I 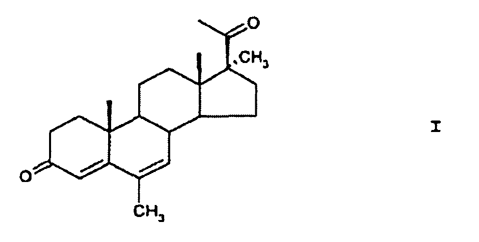 **caractérisé en ce qu'**un composé de formule II 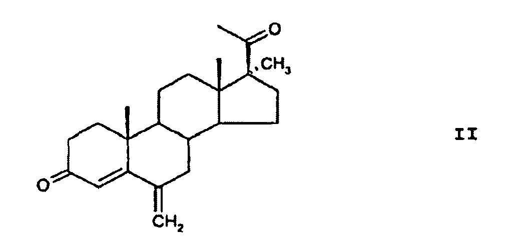 est isomérisé dans un C₁₋₄ alcool ou dans un mélange de tels C₁₋₄ alcools et en présence d'un catalyseur au palladium supporté à une température de 60°C à 95°C et où aucun donneur d'hydrogène n'est ajouté pour former un composé de formule I.

2. Procédé selon la revendication 1, qui est conduit dans le méthanol, l'éthanol, le 2-propanol ou dans un mélange des alcools mentionnés précédemment.

3. Procédé selon la revendication 1, qui est conduit dans l'éthanol.

4. Procédé selon la revendication 1, où du palladium sur oxyde d'aluminium, du palladium sur carbone activé et/ou du palladium sur carbonate de calcium est utilisé comme catalyseur au palladium supporté.

5. Procédé selon la revendication 4, où du palladium à 5 % sur carbone activé est utilisé comme catalyseur au palladium supporté.

6. Procédé selon la revendication 1, où le procédé est conduit à une température supérieure ou égale à 72°C.

7. Procédé selon la revendication 1, où la médrogestone obtenue après l'isomérisation est précipitée sous forme de solide, de préférence sous forme cristalline, après l'addition d'eau.

8. Procédé selon la revendication 7, où la médrogestone précipitée sous forme de solide est dissoute à une température supérieure à la température ambiante par addition de 3,5 - 4,5 unités volumiques d'éthanol, pour une unité massique de médrogestone précipitée sous forme de solide, puis est précipitée de nouveau par refroidissement à la température ambiante ou à une température inférieure à la température ambiante.

9. Procédé selon la revendication 7 ou 8, où la médrogestone obtenue sous forme de solide dans chaque cas après la précipitation est purifiée encore par recristallisation dans le 2-propanol.

10. Procédé selon l'une des revendications 7-9, où la médrogestone obtenue sous forme de solide dans chaque cas est ensuite micronisée.

11. Procédé selon la revendication 1, **caractérisé en ce que** le composé de formule II est obtenu tout d'abord par réduction d'un composé de formule III 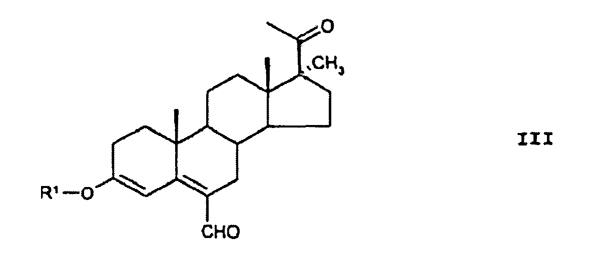 où R¹ est C₁₋₄ alkyle, dans un solvant organique polaire qui est inerte dans les conditions de la réaction ou dans un mélange de solvants avec un hydrure de métal alcalin et l'intermédiaire résultant est ensuite mis à réagir avec un réactif approprié pour cliver le C₁₋₄ alkyléther.

12. Procédé selon la revendication 11, **caractérisé en ce que** le composé de formule III est obtenu par réaction d'un composé de formule IV 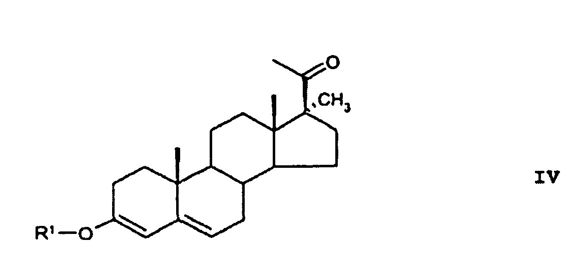 où R¹ est C₁₋₄ alkyle, dans un solvant organique qui est inerte dans les conditions de la réaction ou dans un mélange de solvants avec le diméthylformamide et l'oxychlorure de phosphore.
